# EUROPEAN PATENT APPLICATION

(11) **EP 1 447 065 A1**
(43) Date of publication of application: **18.08.2004**
(21) Application number: 02746131.8
(22) Date of filing: 22.07.2002
(51) Int. Cl.: A61F 13/15

(54) **ABSORBER IN A SHEET FORM AND ABSORBER PRODUCT USING THE SAME**

(30) Priority: 23.07.2001 JP 2001220989
(71) Applicant: Japan Absorbent Technology Institute, Chuo-ku, Tokyo 103-0007 (JP)
(72) Inventor: SUZUKI, Migaku Japan Absorbent Technology Inst., Chuo-ku, Tokyo 103-0007 (JP); SUGIYAMA, Katsuhiko, Kasugai-shi, Aichi 486-0834 (JP); MORI, Kazuyo Livedo Corp. Sadamitsu Plant, Mima-gun, Tokushima 779-4104 (JP)
(74) Representative: Gervasi, Gemma, Dr.
(86) International application number: PCT/JP2002/007384
(87) International publication number: WO 2003/009791

(57) **Abstract**

An absorbent sheet having concave and convex portions on its surface and spontaneously exhibiting a three-dimensional structure in that a concave-convex structure is formed wherein when the difference in level between the concave and the convex portion is A mm in a state where water is not absorbed (dry state) the difference in level between the concave and the convex portion becomes 2A or more after water is absorbed, due to the bulging in parts caused by absorption of water and resultant swelling.

## Description

### Technical Field

The present invention relates to an absorbent product and more specifically to an absorbent sheet to be used in a urine pad, an incontinent product, a diaper and the like.

### Background Art

Conventionally, as means for preventing leakage from the sides of an absorbent product of urine are known such methods as disposing gathers in a three-dimensional structure and folding back an absorbent body to form an anti-leakage wall.

Said method for folding back an absorbent body is effective in that, as urine is absorbed, absorbent polymer contained inside swells and thus the absorbent body is made to stand up to make the firmness of the wall stronger. As the result that the swelling of the absorbent polymer, however, is in all directions, and thus does not stand up changing its shape in any one direction as desired, the standing up of the absorbent polymer is gradual and slow and in many cases urine may overflow before it is checked.

### Disclosure of Invention

An object of the present invention is securely to improve the effect of preventing the leakage of urine, in an absorbent body which may form a leakage prevention wall by standing up as absorbs liquid, by raising the speed of such portion of the absorbent body as forms the leakage prevention wall standing up.

The present invention covers each of the following inventions:

An absorbent sheet having concave and convex portions on its surface and spontaneously exhibiting a three-dimensional structure in that a concave-convex structure is formed wherein when the difference in level between the concave and the convex portion is A mm in a state where water is not absorbed (dry state) the difference in level between the concave and the convex portion becomes 2A or more after water is absorbed, due to the bulging in parts caused by absorption of water and resultant swelling.

In a form of the present invention, a leakage prevention wall may be formed by an absorbent body, whereby in case the absorbent body is applied in an absorbent product, an elastic member for forming a leakage prevention system is no required unlike a conventional absorbent product.

Preferred examples of the above-mentioned invention are given below:

When said difference in level between the concave and the convex portion when no water is absorbed yet is A mm, said difference in level between the concave and the convex portion after water is absorbed becomes 3A mm or more. In the present form of the invention, a leakage prevention wall of a more preferred form may be formed than the form described above.

Said difference in level between the concave and the convex portion when no water is absorbed yet is 0.2 mm~2.0 mm. In the present form of the invention, no feeling of physical disorder is caused by the concavity-convexity when worn.

Said absorbent sheet comprises a P sheet forming an absorbent layer containing super absorbent polymer (SAP) on one side of its substrate sheet, and the content of said SAP inside said absorbent layer is 50 wt.%~ 95 wt.%. In the present form of the invention, a good function of spontaneously providing a three-dimensional structure may be provided for forming a leakage prevention wall.

Said P sheet is 2.0 mm or less thick when no water is absorbed yet. In the present form of the invention, an extremely thin sheet may be obtained for forming a leakage prevention wall.

On said P sheet are formed absorbent layers on either side of said substrate sheet formed in bands at given intervals. In the present form of the invention, an absorbent sheet may be obtained which may be well processed.

On said P sheet a Q sheet is laminated for supporting and fixing said P sheet. In the present form of the invention, an absorbent sheet which is dimensionally stable may be obtained.

Said P sheet and said Q sheet are bonded together intermittently at given intervals. In the present form of the invention, a sheet having such concave and convex portions as uniformly bulge by absorbing liquid and accordingly swelling may be obtained.

The bonding spacing between said P sheet and said Q sheet is 5 mm or more. In the present form of the invention, an absorbent sheet which may be well processed and have concave and convex portions capable of forming a state where a firm stand-up is formed may be obtained.

Said P sheet is bonded to said Q sheet at the portion where no absorbent layers are formed. In a mode of the present invention an absorbent sheet excellent in processability and having convex and concave portions forming a firm standing up condition may be obtained.

Said concave and convex portions are formed by folding back said absorbent sheet. In the present form of the invention, a leakage prevention wall may be formed which stands up much due to the absorbent sheet being bent back by absorbing of liquid and accordingly swelling.

Said concave and convex portions are formed by bonding to said Q sheet intermittently at given intervals while said P sheet is given some slackening. In the present form of the invention, an absorbent sheet having the concave and the convex portion which are more different in level between the two portions before and after liquid is absorbed may be obtained.

Said P sheet is bonded to said Q sheet at the portion where no absorbent layers are formed. In a mode of the present invention an absorbent sheet excellent in processability and having convex and concave portions forming a firm standing up condition may be obtained.

On to said P sheet a laminated structure is formed where an absorbent sheet with an absorbent layer formed containing said SAP on the surface of said absorbent substrate sheet is laminated at least in one layer and bonded intermittently to each other, and said P sheet and said Q sheet are bonded to each other intermittently. In the present form of the invention, an absorbent sheet having such concave and convex portions as are further more different in level before and after liquid is absorbed may be obtained.

Said absorbent layer contains super absorbent polymer (SAP) and its bonding agent, i.e., microfibrillated cellulose (MFC). In the present form of the invention, an absorbent sheet where SAP hardly falls off may be obtained.

Said substrate sheet of said P sheet comprises hydrophilic and water diffusive wood pulp, rayon-based non-woven fabric, cotton-based non-woven fabric, or at least one kind of non-woven fabrics made hydrophilic selected from a group consisting of polyethylene, polypropylene, polyethylene terephthalate and polyvinyl alcohol fibers. In the present form of the invention, since the P sheet is hydrophilic and water diffusive, an absorbent sheet which is excellent in absorbency and diffusion may be obtained.

Said Q sheet comprises at least one kind of hydrophobic non-woven fabrics selected from a group consisting of a spun bonded or a spun bonded/melt blown/spun bonded composite comprising polyethylene, polypropylene, polyethylene terephthalate and polyvinyl alcohol fibers. In the present form of the invention, since the Q sheet is made of a flexible material, an absorbent sheet may be obtained which is apt to fit the body of a wearer.

Said Q sheet comprises a film or an apertured film selected from a group consisting of polyethylene, polypropylene, polyethylene terephthalate and polyvinyl alcohol types or a composite of any of such films with a non-woven fabric. In the present form of the invention, since the Q sheet is made of a rigid material, an absorbent sheet may be obtained which has a good tendency to stand up.

In addition, the present invention is an absorbent product comprising a liquid permeable top sheet, a liquid impermeable leakage prevention sheet and an absorbent body for absorbing and retaining liquid as disposed between the sheets and having a guarding function against liquid on both side edges and/or on the flange ends of the front body and the back body, wherein said absorbent body comprises an absorbent sheet having either function of spontaneously becoming three-dimensional. In the present form of the invention, since a leakage prevention wall may be formed by means of an absorbent body, no elastic member for forming a leakage prevention system is required unlike a conventional absorbent product and an absorbent product excellent in the leakage prevention effect on the side edges and the peripheral edge may be obtained.

Preferred forms of the above-describe invention are given below:

Said absorbent body is sectioned into the center portion and the side portions on both sides, and said side portions on both sides have a laminated structure having a difference in thickness of 1.0 mm or less between both the side portions and the center portion when no liquid is absorbed yet and is made to stand up so that said both side portions have a difference in thickness of 2.0 mm or more compared with the center portion when the absorbent body absorbs liquid and swells whereby the absorbent body functions as a side guard bank. In the present form of the invention, an absorbent body is suitable for absorbent products such as feminine hygiene products, liners for urine and lightly incontinent pads requiring no highly absorbing speed and an absorbent product having a capability of preventing leakage from the side edges may be obtained.

Said absorbent body is sectioned into the center portion and the side portions on both sides, and said side portions on both sides have a folded structure and have a difference in thickness of 2.0 mm or less between both the side portions and the center portion when no liquid is absorbed yet and is made to stand up so that said both side portions have a difference in thickness of 5.0 mm or more compared with the center portion when the absorbent body absorbs liquid and swells whereby the absorbent body functions as a three dimensional side guard bank. In the present form of the invention, an absorbent body is suitable for absorbent products such as disposable diapers requiring a highly absorbing speed and an absorbent product having a capability of preventing leakage from the side edges may be obtained.

Said absorbent body is sectioned into the peripheral edge portions on both sides of the front and the back body and the central portion, and said side portions on both sides have a laminated structure having a difference in thickness of 1.0 mm or less between both the peripheral edge portions and the center portion when no liquid is absorbed yet and is made to stand up so that said both side portions have a difference in thickness of 2.0 mm or more compared with the center portion when the absorbent body absorbs liquid and swells whereby the absorbent body functions as a side guard bank. In the present form of the invention, an absorbent body is suitable for absorbent products such as feminine hygiene products, liners for urine and lightly incontinent pads requiring no highly absorbing speed and an absorbent product having a capability of preventing leakage from the peripheral edges may be obtained.

Said absorbent body is sectioned into the peripheral edge portions on both sides of the front and the back body and the central portion, and said side portions on both sides have a folded structure and have a difference in thickness of 2.0 mm or less between both the side portions and the center portion when no liquid is absorbed yet and is made to stand up so that said both peripheral edge portions have a difference in thickness of 5.0 mm or more compared with the center portion when the absorbent body absorbs liquid and swells whereby the absorbent body functions as a side guard bank. In the present form of the invention, an absorbent body is suitable for absorbent products such as disposable diapers requiring a highly absorbing speed and an absorbent product having a capability of preventing leakage from the side edges may be obtained.

On the back body of said absorbent body a member for guiding solid human waste is formed comprising a concave and convex structure having a difference in thickness of 2 mm or less when no liquid is absorbed and a difference in thickness of 5 mm or more when the absorbent body absorbs liquid and swells. In the present form of the invention, since a member for guiding solid human waste is formed on the back body, an absorbent product such as a disposable diaper wherein the body of a wearer is made to less contacts with solid human wastes.

In the center region of said absorbent body in a longitudinal direction, a convex portion structure is formed which has a difference in level of 2 mm or less from its surrounding region when no liquid is absorbed and has a difference in level of 5 mm or less from its surrounding region when the absorbent body absorbs liquid and swells whereby the absorbent body is made to stand up so that the absorbent body is made to closely contact with the discharging parts. In the present form of the invention, since the discharging parts are made to contact with the absorbent body, an absorbent product may be obtained wherein liquid human wastes may be absorbed at an intended position of the absorbent body.

### Brief Description of Drawings

Fig. 1 is an illustrative oblique perspective figure of an absorbent sheet according to the present invention.
Fig. 2 is a cross sectional view of the absorbent sheet shown in Fig. 1 before liquid is absorbed.
Fig. 3 is a cross sectional view of the absorbent sheet shown in Fig. 1 after liquid is absorbed.
Fig. 4 is a cross sectional view of another example of an absorbent sheet according to the present invention before liquid is absorbed.
Fig. 5 is a cross sectional view of the absorbent sheet shown in Fig. After liquid is absorbed:
Fig. 6 is a cross sectional view of an example of an absorbent sheet according to the present invention before liquid is absorbed.
Fig. 7 is a cross sectional view of the absorbent sheet shown in Fig. 6 after liquid is absorbed.
Fig. 8 is a cross sectional view of another example of an absorbent sheet according to the present invention before liquid is absorbed.
Fig. 9 is a cross sectional view of the absorbent sheet shown in Fig. 8 after liquid is absorbed.
Fig. 10 is a cross sectional view of another example of an absorbent sheet according to the present invention before liquid is absorbed.
Fig. 11 is a cross sectional view of the absorbent sheet shown in Fig. 10 after liquid is absorbed.
Fig. 12 is a cross sectional view of a still other example of an absorbent sheet according to the present invention before liquid is absorbed.
Fig. 13 is a cross sectional view of the absorbent sheet shown in Fig. 12 after liquid is absorbed.
Fig. 14 is a plane view of a tape-type disposable diaper using an absorbent sheet according to the present invention.
Fig. 15 is a cross sectional view of the disposable diaper taken along the line V to V' shown in Fig. 14.
Fig. 16 is a cross sectional view of the disposable diaper taken along the line X to X' shown in Fig. 14.
Fig. 17 is a plane view of another example of a tape-type disposable diaper using an absorbent sheet according to the present invention.
Fig. 18 is a cross sectional view of the disposable diaper taken along the line Y to Y' shown in Fig. 17.
Fig. 19 is a plane view of a urine pad using an absorbent sheet according to the present invention.
Fig. 20 is a cross sectional view of the urine pad taken along the line Z to Z' shown in Fig. 19.

### Best Mode for Carrying Out the Invention

An absorbent sheet according to the present invention has small concave and convex portions formed on the surface. These concave and convex portions are characterized in that they do not have differences in levels so large as to give rise to any uncomfortable feeling to a wearer before liquid is absorbed, but, once liquid is absorbed, such differences in level are made suddenly large as liquid is absorbed. Such differences in level are formed by using an absorbent sheet having an absorbent layer containing a lot of super absorbent polymer (SAP).

An absorbent sheet according to the present invention involves an embodiment where, by virtue of concave and convex portions formed on the surface of the sheet, the convex portions are formed by folding the sheet and the flat portions not as folded are made concave portions.

In addition, another embodiment is involved where, for forming concave and convex portions, convex portions are formed by laminating another absorbent sheet onto an absorbent sheet and such portions where an absorbent sheet is not thus laminated are made concave portions.

Furthermore, an embodiment is also contained where for making differences in level between concave and convex portions by causing an absorbent sheet to swell in much different degrees, two elements different in expansion coefficient are stuck together by means of which effects of some bending being caused in a given direction different by differences in deformation caused by liquid being absorbed.

A fundamental principle underlying such structure of an absorbent sheet may be the same as a bimetal principle applied as an electric contact and a crimp generating principle as seen in a two component side by side fiber.

In case an absorbent sheet having concave and convex portions according to the present invention is used to make an absorbent product, the absorbent sheet may be made to serve as a leakage preventing wall to prevent leakage from the side edges and peripheral edges of the absorbent product. Since the absorbent sheet functions as a leakage preventing wall, a three-dimensional gathers need not be formed using an elastic material as in a conventional absorbent product.

An absorbent sheet according to the present invention is characterized in that, when the difference in level between concave and convex portions is made A mm with liquid yet to be absorbed (in a dry state), the difference in level between the concave and convex portions become 2A mm or more, preferably 3A mm or more, due to bulging in parts after liquid is absorbed when physiological saline (hereinafter "liquid is (or being) absorbed" meaning "physiological saline is (or being) absorbed") is absorbed so that the absorbent sheet is expanded. Also, the difference (A) in level between the concave and convex portions before liquid is yet to be absorbed is preferably 0.2 mm ∼ 2.0 mm. If such difference is less than 0.2 mm, the effect of the absorbent sheet as a leakage preventing wall becomes less than desired, and, on the other hand, if such difference in level is more than 2.0 mm, uncomfortable feeling is caused to a wearer due to the large difference in level.

Furthermore, the thickness of an absorbent sheet before liquid is absorbed is preferably 2.0 mm or less. If such thickness becomes more than 2.0 mm, the concave and convex portions cannot appropriately be processed.

An absorbent sheet according to the present invention is preferably made with two kinds of sheets different in expansion coefficient in water being stuck together in a not expanded state. In other words, by sticking together a P sheet of high expandability with an absorbent layer containing a high concentration of SAP formed on one side of a substrate sheet and a Q sheet holding and fixing said P sheet containing little or non SAP thereby a swelling and expanding function is provided inherent in SAP when water is absorbed so that a three-dimensional structure may be imparted to an absorbent sheet.

In the present invention, said three-dimensional structure being imparted is called as a spontaneous three-dimensional structure being imparted since it is imparted spontaneously as accompanied by the water swelling phenomenon.

The forms of such spontaneous three-dimensional structure depend much on the nature and shape of the P sheet, the nature and shape of the Q sheet to be bonded to the P sheet and the bonding state of both sheets.

In an absorbent sheet according to the present invention, the forms of such spontaneous three-dimensional structure are classified into two groups as follows:

In the first embodiment, P sheets and Q sheets are bonded together intermittently at certain intervals. In this embodiment, as a P sheet a substrate sheet is used where on the substrate sheet absorbent-layer-existent-regions and absorbent-layer-non-existent-regions are alternately formed to make the sheet flexible and easy to bond, and as a Q sheet, a sheet of no swelling and of dimensional stability is used, and an absorbent sheet is thus advantageously' made where in the regions of no absorbent layer in existence of the P sheet, the P sheet is bonded to the Q sheet intermittently at certain intervals by means of such methods as thermal fusion.

If such absorbent sheet is made to absorb water to be swollen, the water absorbing and swelling capacity is restricted to the bonding range and at the same time the sheet is made to bulge due to its ease to deform and by virtue of tonicity caused thereby the sheet is caused to be much three-dimensional.

Also, a P sheet and a Q sheet are bonded at the interval of 5 mm or more, preferably, of 5 ~ 50 mm. If the bonding interval is less than 5 mm, the bonding interval becomes too narrow thereby its water absorbing and swelling capacity is impeded' and thus the function of making a spontaneous three-dimensional structure becomes insufficient. On the other hand, the bonding interval gets wider, more than 50 mm, the bulging capacity caused by the water absorbing and swelling is dissipated so that a strong three-dimensional structure cannot be obtained.

Furthermore, a P sheet and a Q sheet may be bonded not only as they are stuck together as extended but also, with the P sheet being molded as folded, slackened or channeled to be stuck together with the Q sheet, sufficient space for swelling is provided thereby even a small swelling capacity may make the sheet easier to be of three-dimensional structure.

Also, by laminating at least one layer of a substrate sheet made water absorbent with an absorbent layer containing SAP formed on its surface to said P sheet thereby a laminated structure where both sheets are bonded in parts is formed in which said P sheet and said Q sheet are bonded to each other in parts so that the sheets are thus made to be easy to expand.

A second embodiment of a spontaneous three-dimensional structure may be that a P sheet and a Q sheet are continuously bonded and folded in that the P sheet side is made to come inside so that such folded part is made a convex portion. In other words, an absorbent sheet deforms as warped toward a Q sheet even if a P sheet absorbs liquid to swell since the liquid absorbing and swelling capability of the Q sheet is small, and thus if beforehand the absorbent sheet is folded with the P sheet made to come inside, the absorbent sheet with the difference in level between the concave and convex portions which is small before liquid is absorbed may be made to provide a large leakage prevention wall with the folded portion getting warped as liquid being absorbed. In this case, if both sheets are bonded intermittently, the effect becomes low so that the sheets are preferably bonded continuously.

Also, if as a P sheet a sheet with an absorbent-layer-existent-region and an absorbent-layer-non-existent-region formed alternately on a substrate sheet is used and the P sheet is folded in the absorbent-layer-non-existent-region, an absorbent sheet which can be processed appropriately may be obtained.

The forms of folding an absorbent sheet may be such that the sheet is folded with its both side edge being made to come inside, or the sheet is folded with its both side edges being made to come inside and then is made to have a Z-shaped cross sectional form with the folded portion folded back midway.

As a substrate sheet constituting a P sheet according to the present invention, a sheet may be used comprising wood pulp, rayon-based non-woven fabric, or cotton-based non-woven fabric, which is hydrophilic and diffusive. Also, spun bonded or spun bonded/melt blown/spun bonded composite non-woven fabric comprising polyethylene-based, polypropylene-based, polyethylene-terephthalate-based, polyvinyl-alcohol-based fibers may be used after they are treated to be hydrophilic.

An absorbent layer constituting a P sheet contains preferably SAP of 50 wt%~95wt%. If the SAP content is less than 50wt%, a spontaneous three-dimensional structure may not be sufficiently obtained. If the SAP content is more than 95wt%, SAP may be likely to fall off so that an absorbent sheet cannot be appropriately processed.

As the coating materials constituting an absorbent layer, such coating materials as contains SAP and microfibrillated fiber (MFC) as the bonding agent of SAP is preferable. For example, a P sheet formed by applying this type of coating to a non-woven fabric may be made extremely thin so that it may extremely easily be folded and laminated. Also, a P sheet may be used which is made with 2 or more layers laminated.

A Q sheet which may be used for the present invention is a sheet containing little or no SAP. The Q sheet may be at least one type of hydrophobic non-woven fabrics selected from spun bonded and spun bonded/melt blown/spun bonded composites comprising polyethylene-based, polypropylene-based, polyethylene-terephthalate-based, and polyvinyl-alcohol-based fibers. Furthermore, film or an apertured film or zygote of any such film and a non-woven fabric selected from polyethylene-based, polypropylene-based, polyethylene-terephthalate-based, and polyvinyl-alcohol-based fibers.

If as a material constituting a Q sheet such sheet material as is flexible and easy to deform is used, a Q sheet also deforms according to the deformation and bulging of a P sheet so that an absorbent sheet fitting the bodily contour of a wearer may be obtained. Also, if a sheet material which is dimensionally stable and rigid is used as a material constituting a Q sheet, an absorbent sheet obtained will not be fitting the bodily contour of a wearer although the deformation and bulging of a P sheet is likely to happen. In general, wrapping tissue for an absorbent body or a back sheet used in an absorbent product are advantageously used as a Q sheet, and an apertured film having a relatively high rigidity may be disposed as a Q sheet between a back sheet and a P sheet.

A P sheet and a Q sheet may be bonded by thermal fusion such as a thermal seal, by adhesive bonding such as hot melt or by a polyethylene sheet disposed in between.

Said absorbent sheet according to the present invention may be utilized in or for a variety of absorbent products, and as the sheet is applied in a baby diaper and an incontinent diaper for an adult, gathering function such as waist gather may be realized by giving an absorbent structure form without using any elastic member.

An absorbent sheet according to the present invention may be used as an absorbent body used in an absorbent product having a liquid permeable top sheet, a liquid impermeable leakage preventing sheet and an absorbent body disposed between both sheets for absorbing and holding liquid, which absorbent sheet absorbs bodily fluids and at the same time functions as a guard member for preventing liquid leakage at the side edges and/or the peripheral edges of the front and the back body of the absorbent product.

In other words, the absorbent body is sectioned into a middle portion and side edge portions. Said side edge portions have each a laminated structure having a difference in level in terms of thickness of 1.0 mm or less as compared with said central portion when no liquid is absorbed yet, and function as a side guard bank as said edge portions are made to stand up when they absorb liquid to swell with difference in level in terms of thickness of 2.0 mm or more as compared with said central portion.

In addition, an absorbent sheet according to the present invention may be made as its absorbent body is sectioned into a central portion and side portions. Each of said side portions has a folded structure and, when no liquid is absorbed yet, has a difference in level being 2 mm or less thicker than said central portion and, as it absorbs liquid to swell, it is made to stand up as has a difference in level in terms of thickness of 5 mm or more compared with said central portion so that it functions as a three dimensional side guard bank.

Furthermore, an absorbent body may be sectioned into the peripheral edge portions of the front and the back body and the central portion. Each of said peripheral edge portion has a laminated structure having a difference in level in terms of thickness of 1.0 mm or less when no liquid is absorbed compared with said central portion and, as it absorbs liquid to swell, it is made to stand up as has a difference in level in terms of thickness of 5 mm or more compared with said central portion so that it functions as a three dimensional end guard bank.

Also, an absorbent body may be sectioned into the peripheral edge portions of the front and the back body and the central portion. Each of said peripheral edge portion has a folded structure having a difference in level formed in terms of thickness of 2.0 mm or less when no liquid is absorbed compared with said central portion and, as it absorbs liquid to swell, it is made to stand up as has a difference in level in terms of thickness of 5 mm or more compared with said central portion so that it functions as a three dimensional side guard bank.

An absorbent body thus having a laminated structure and a folded structure in both side edge portions and both edge portions may be used as it has such laminated structure and such folded structure as formed in the above-mentioned manner.

Also, an absorbent body having a concave portion between two convex portions from the crotch region extending to the back body region and having a concave-convex structure as formed having a difference in level in terms of thickness of 2.0 mm or less when no liquid is absorbed yet and a difference in level when it absorbs liquid to swell is used thereby a concave-convex structure which may serve a guiding member for solid human waste at the back body portion of an absorbent product is formed so that the direct contact of the skin to the solid human waste is made less.

Furthermore, an absorbent body is used in which its longitudinal central region is made to stand up as has a difference in level in terms of thickness of 2 mm or less compared with its peripheral area when no liquid is absorbed yet and as has a difference in level in terms of thickness of 5 mm or more compared with its peripheral area thereby it may be utilized for an absorbent product having as formed a convex portion structure that the central region is made to closely contact the discharging area.

Hereunder, the present invention Will be explained with reference to the drawings showing examples of the present invention, and the present invention will not be limited to these examples.

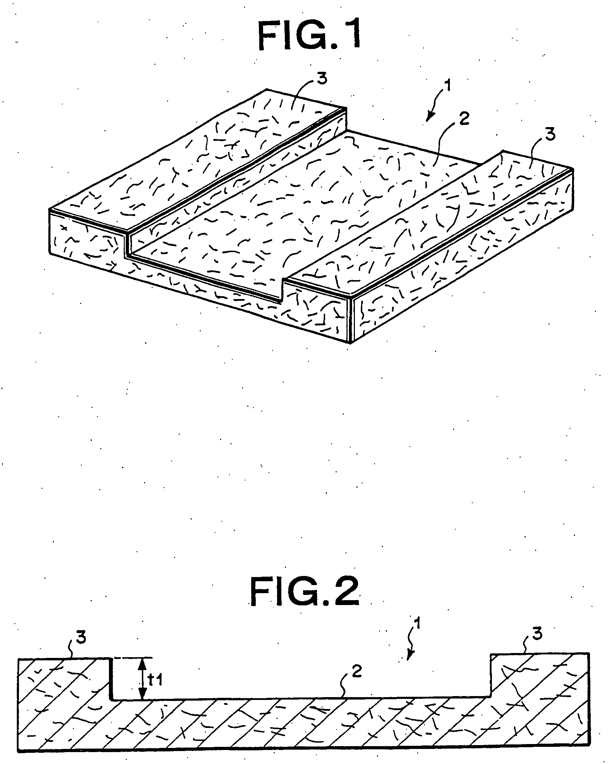

Fig. 1 illustrates an oblique perspective figure of a state of an absorbent sheet 1 according to the present invention before no liquid is absorbed and its convex portions are shown by 2 and concave portions are shown by 3.

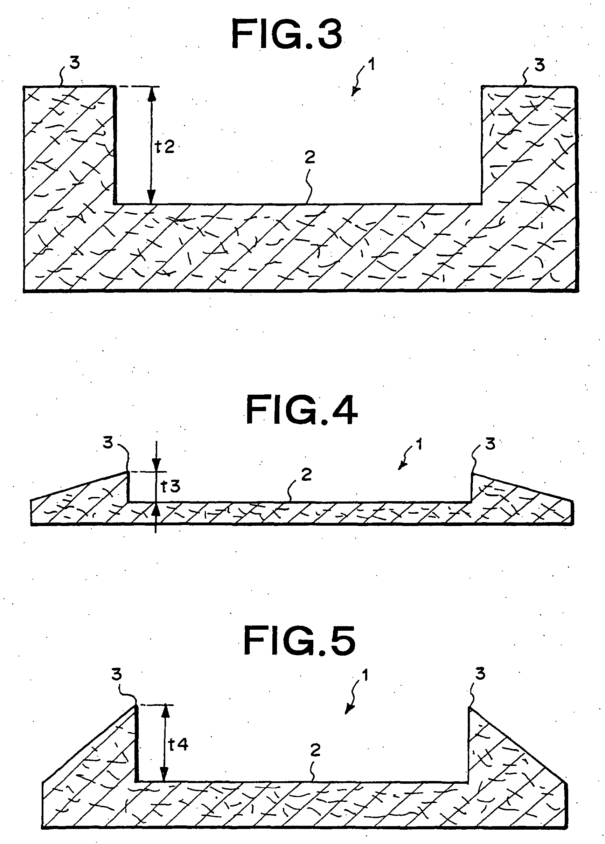

Figs. 2 and 3 show a cross sectional view of an absorbent sheet shown by Fig. 1 before and after liquid is absorbed by such absorbent sheet. In Fig. 2, a difference in level between the concave portion 2 and the convex portion 3 after liquid is absorbed is shown by t 2. An absorbent sheet according to the present invention is characterizes in that, if a difference in level between a convex portion and a concave portion before liquid is absorbed yet is A mm, the difference in level between the convex portion and the concave portion after liquid is absorbed will be 2A mm or more. In other words, the relation between t1 and t2 shown in Figs. 2 and 3 is given by a formula 2 x t1 ≦ t2.

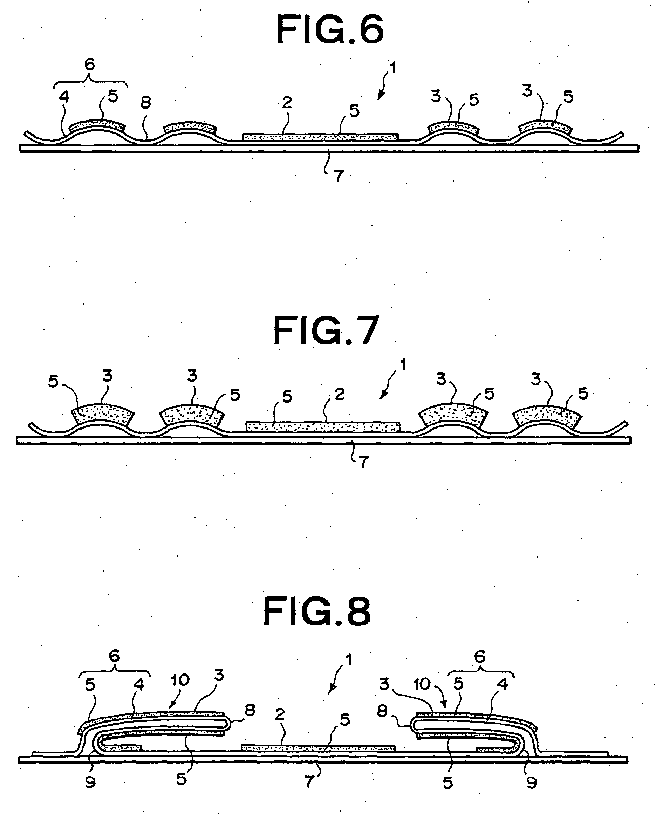

Figs. 4 and 5 are cross sectional views of other forms of the convex and concave portions of an absorbent sheet according to the present invention before and after liquid is absorbed. In Fig.4, a difference in level between the convex portion 3 whose top is inclined and the concave portion 2 is given as t3, while, in Fig. 5, a difference in level between the convex portion 3 and the concave portion 2 after liquid is absorbed is given as t4, and the relation between 13 and t4 is given by the formula 2 x t3 ≦ t4.

Figs. 6 and 7 are cross sectional views of specific examples as formed of a convex and a concave portion of an absorbent sheet according to the present invention.

Fig. 6 indicates in a cross sectional view the state of an absorbent body 1 of the absorbent sheet before liquid is absorbed wherein a P sheet comprising absorbent layers 5 as formed on a substrate sheet 4 and a Q sheet are bonded. In other words, the P sheet 6 is bonded to the Q sheet 7 to form convex portions 3 in the area 8 where no absorbent layers are not formed as slackening is given in the area where absorbent layers are formed in the region of the P sheet's side edge portions, and, furthermore, the area of the central region where absorbent layers 5 are formed is bonded to the P sheet to form the convex portions 2.

Fig. 7 shows a state of the absorbent sheet 1 after liquid is absorbed where the absorbent layers 5 of the P sheet 6 are all thickened as they absorb to swell, and in particular the convex portions 3 are much bulged as warped upward by the action of expansion of the absorbent layers 5 resulting in the difference in level between the convex portions 3 and the concave portions2 being increased to more than two times as large as that before liquid is absorbed.

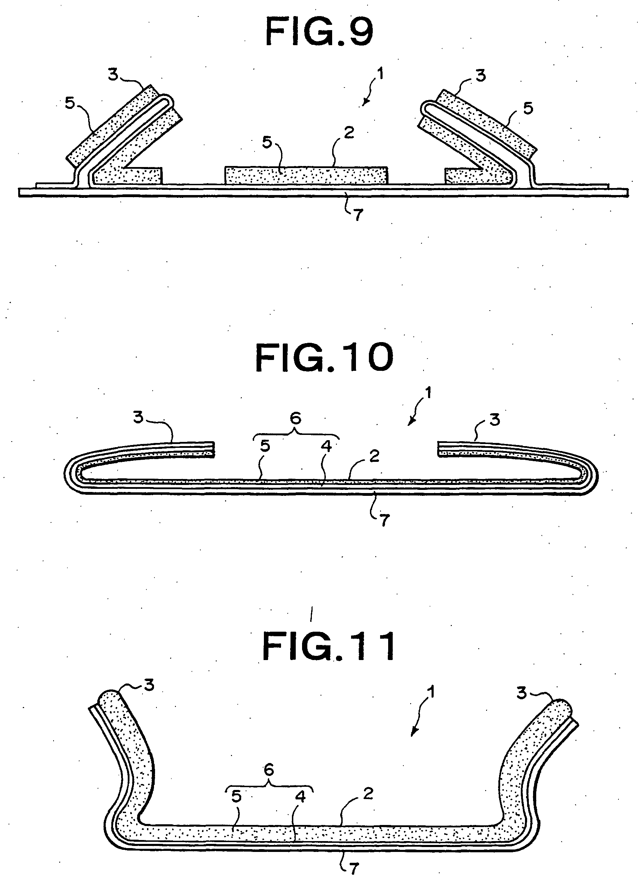

Figs. 8 and 9 are cross sectional views of other forms of the convex portions and the concave portions of an absorbent sheet according to the present invention.

Fig. 8 indicates in a cross sectional view the state of the absorbent sheet 1 before liquid is absorbed showing the state the P sheet 6 comprising absorbent layers 5 as formed intermittently on the substrate sheet 4 and the Q sheet 7 are bonded. In other words, the P sheet is folded in its side edge region with the area 8 where no absorbent layers are not formed between the two absorbent layers 5 as the folding line as the back sides of the neighboring absorbent layers 5 are made to contact with each other and a folding portion 10 is formed as the P sheet in its bottom edge portion 9 is bonded to the Q sheet 7, and then the convex portions 3 are formed as the folding portion 10 is folded back inward. In case the convex portions 3 of this shape are formed, as shown in Fig. 8, out of the bottom portion 9 of the folding portion 10 the bottom portion located inside the absorbent body preferably contains absorbent layers 5.

Furthermore, the concave portions 2 are formed as the central region of the P sheet where no absorbent layers 5 are formed is bonded to the Q sheet.

Fig. 9 indicates the state of the absorbent sheet 1 after liquid is absorbed where all the absorbent layers 5 of the P sheet 6 have absorbed water and thus are swollen and thickened, and in particular, the convex portions 3 are swollen and are made to stand up from the bottom edge portion, and the difference in level between the convex portions 3 and the concave portions 2 before and after liquid is absorbed easily gets two times or more as much as that before liquid is absorbed and, thus, the P sheet 6 is made to further function as a leakage preventing wall.

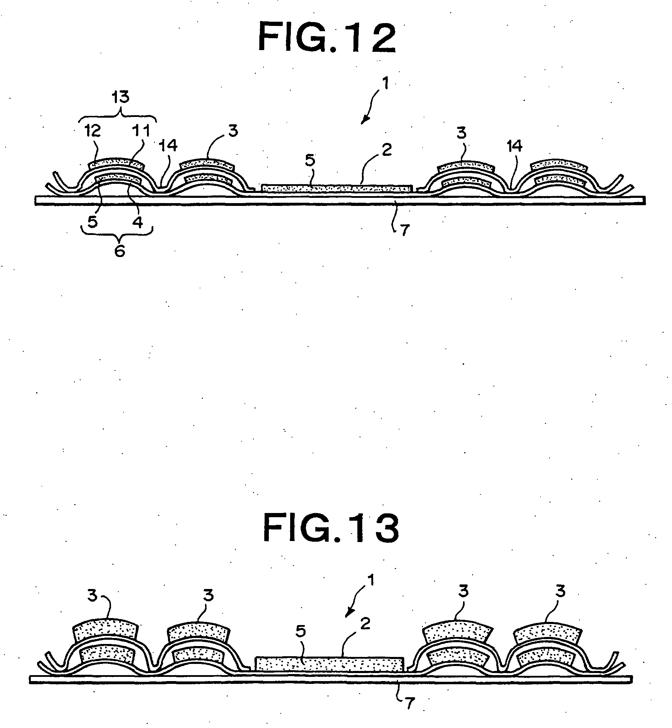

Figs. 10 and 11 are cross sectional views of still other forms of the convex portions and the concave portions of an absorbent sheet according to the present invention.

Fig. 10 indicates in a cross sectional view the state of the absorbent sheet before liquid is absorbed where the P sheet 6 comprising absorbent layers formed all over the substrate sheet 4 and the Q sheet are bonded to each other. In other words, the absorbent sheet 1 is formed as the P sheet 6 and the Q sheet 7 are bonded in an integrated form, and, further, convex portions 3 are formed as the side edge portions of the absorbent sheet 1 are folded inward leaving the central region of the absorbent sheet as the concave portions 2.

Fig. 11 indicates the state of the absorbent sheet 1 after liquid is absorbed where the absorbent layers 5 of the P sheet 6 have absorbed water and thus are swollen and thickened, and, in particular, the folded portions constituting the convex portions 3 are made to stand up, and the difference in level between the convex portions 3 and the concave portions 2 before and after liquid is absorbed easily gets two times or more as much as that before liquid is absorbed and, thus, the P sheet 6 is made to further function as a leakage preventing wall.

Figs. 12 and 13 are cross sectional views of still further other examples of concave and convex portions of an absorbent sheet according to the present invention.

Fig. 12 indicates in a cross sectional view the state of the absorbent sheet 1 before liquid is absorbed where in both side edge portions the P sheet 6 as constituted by the absorbent sheet as laminated and the Q sheet 7 are bonded. In other words, in the absorbent sheet 1 the side edge portions of the P sheet 6 of the absorbent sheet shown in Fig. 6 are laminated with the absorbent sheet 13 comprising the absorbent layers 12 formed intermittently on the substrate sheet 11 and bonded to the P sheet 6 in the area 14 where no absorbent layers are formed so that the convex portions 3 are formed in the laminated portion and the concave portions 2 are made of the central portion where the absorbent layers are formed.

Also, in this case, the P sheet 6 and the absorbent sheet 13 may be formed of the same material or a different material.

Fig. 13 indicates the state of the absorbent sheet 1 after liquid is absorbed where the absorbent layers have absorbed liquid to be swollen so that a bulky leakage preventing wall may be formed.

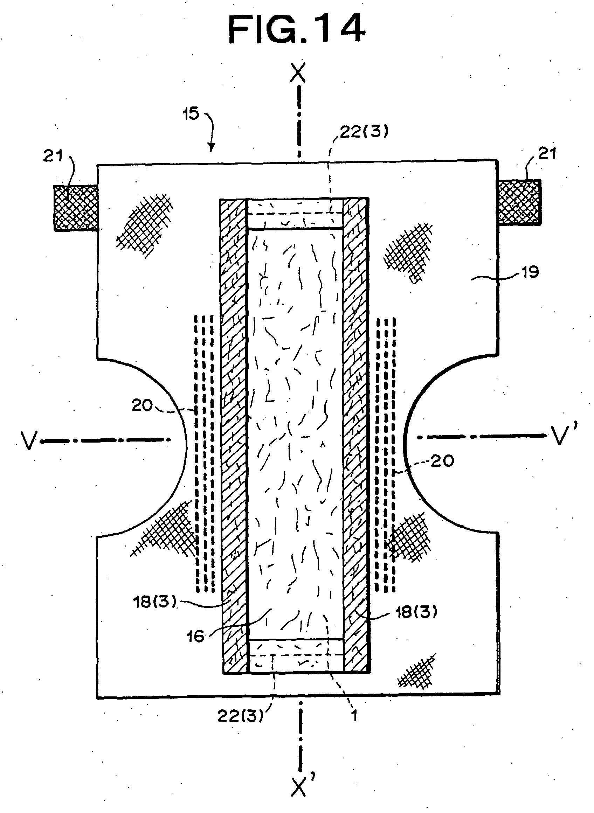

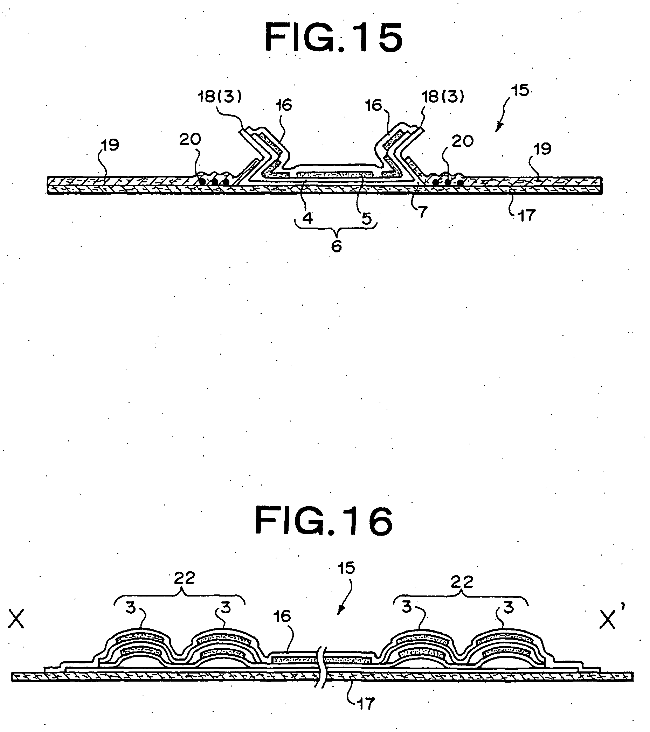

Fig. 14 indicates in a plane view a tape type disposable diaper using an absorbent sheet according to the present invention, Fig. 15 indicates in a cross sectional view the state of the diaper taken along the line V to V' shown in Fig. 14, and Fig. 16 indicates in a cross sectional view the side edge portions of the front and the back body of the disposable diaper taken along the line X to X' shown in Fig. 14.

The tape type disposable diaper 15 is such that an absorbent sheet 1 is disposed in the longitudinal central portion of a wide liquid impermeable leakage preventing sheet 17, a liquid permeable top sheet 16 is disposed as covering the absorbent sheet 1, a side sheet 19 comprising a non-woven fabric is disposed on the leakage preventing sheet 17 in the side edges of the absorbent sheet 1, further in between the side sheet 19 and the leakage preventing sheet 17 an elastic member 20 for surrounding the legs is disposed and formed, and a fastening tape 21 is attached on both sides of the back body.

Also, as shown in Fig. 14, convex portions are formed serving as a side leakage preventing bank 18 and an edge leakage preventing bank 22 on both sides of the absorbent sheet 1 and on the peripheral edge of the back body, respectively.

Also, in this example, as shown in Fig. 15, the absorbent sheet 1 is formed as a P sheet 6 comprising absorbent layers 5 formed intermittently on a substrate sheet 4 and a Q sheet 7 are bonded to each other, and convex portions 3 serving as a side leakage preventing bank as both side portions folded in a Z-form.

Also, as shown in Fig. 16, convex portions 3 are formed in a laminated structure as shown in Fig. 12 and serving as a peripheral leakage preventing bank 22 on both peripheral edges of the front and the back body of the absorbent sheet 1.

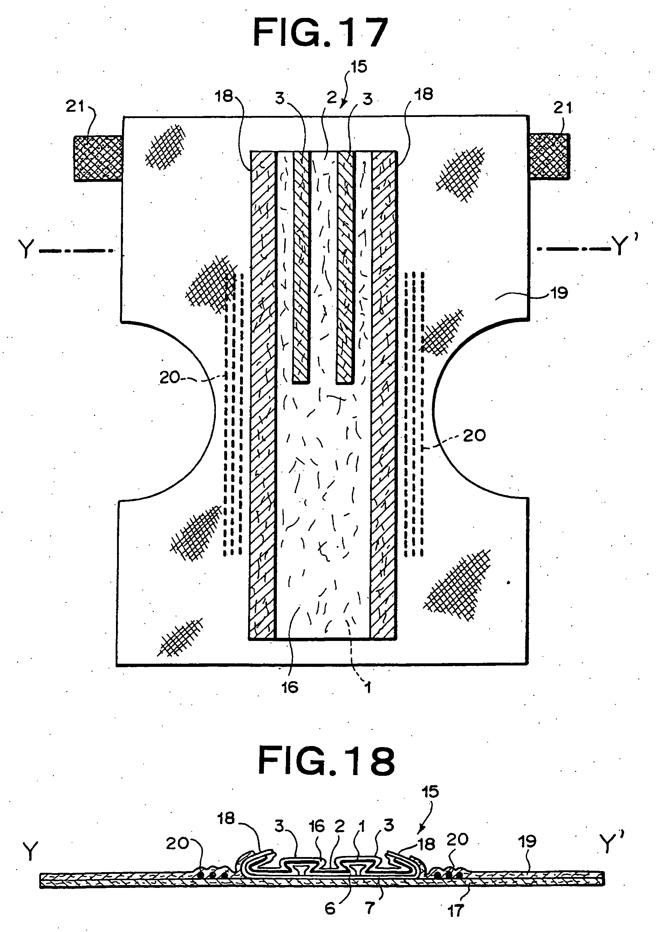

Fig. 17 is a plane view showing another example of a tape type disposable diaper using an absorbent sheet according to the present invention, and Fig. 18 is a cross sectional view showing the state of the diaper taken along the line Y to Y' shown in Fig. 17.

The basic structure of the tape type disposable diaper 15 is the same as that shown in Fig. 2, but, as shown in Fig. 17, a convex and concave structure serving as a solid waste guiding portion comprising two convex portions 3 formed in the central region of the back body of the absorbent sheet1 and one concave portion 2 formed between the two convex portions 3.

In this case, as shown in Fig. 18, the convex portions 3 of the convex and concave portion structure are formed with the surface made flat as the P sheet 6 is bonded to the Q sheet 7 in a condition that the central region of the P sheet 6 is folded in a Ω form in two positions.

By using an absorbent sheet 1 having such convex and concave portion structure, when urine and solid waste are discharged, the convex portions 3 are bulged to make the difference in level larger between the convex portions and the concave portions and solid waste is guided into the concave portions 2 so that solid waste less sticks to the buttocks of a wearer.

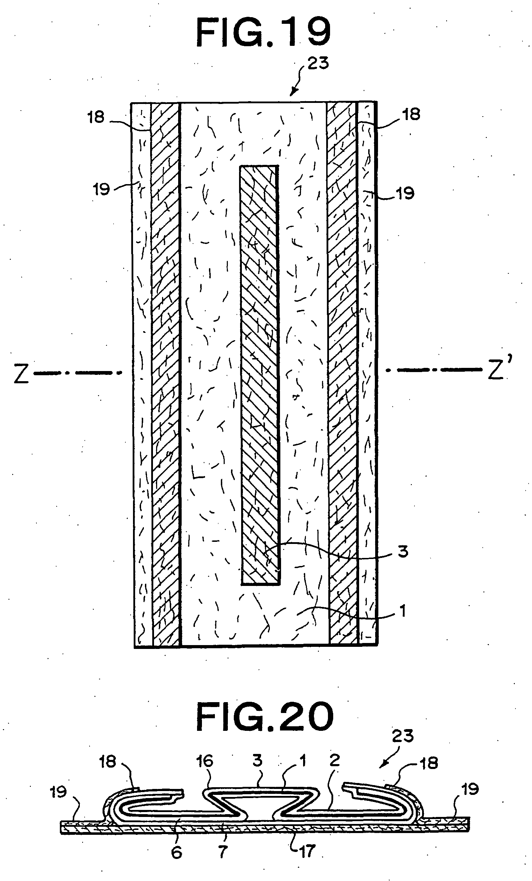

Fig. 19 is a plane view of a urine pad using an absorbent sheet according to the present invention, and Fig. 20 is a cross sectional view indicating the state of the urine pad as shown in Fig. 19 taken along the line Z to Z'.

The urine pad 23 is formed as an absorbent sheet 1 is disposed on the top of a liquid impermeable leakage preventing sheet 17, a liquid permeable top sheet 16 is disposed as it covers the absorbent sheet 1 and, further, a side sheet 19 comprising a non-woven fabric is disposed on the leakage preventing sheet 17 on the side edges of the absorbent sheet 1.

In this example, as shown in Fig. 19, convex portions 3 are formed in the central portion in the longitudinal direction of the absorbent sheet 1 constituting a urine pad 23, which convex portions when they absorb liquid swell and are made to stand up so that they get in close contact with the discharging areas.

In this case, as shown in Fig. 20, the convex portions 3 are formed with the surface made to be flat as the P sheet while its central portion is folded in an Ω form is bonded to the Q sheet.

### Industrial Applicability

Absorbent sheets according to the present invention and absorbent products using such absorbent sheets are suitable for making urine pads, incontinent products, diapers and the like.

## Claims

1. An absorbent sheet **characterized in that** it has convex and concave portions on its surface and has a spontaneous three-dimensional structure, wherein when a difference in level between the convex portions and the concave portions is A mm before a liquid is absorbed, the difference in level between the convex portions and the concave portions becomes 2A mm or more after it absorbs the liquid to swell so that it bulges in parts.

2. An absorbent sheet according to claim 1, wherein when said difference in level between the convex portions and the concave portions is A mm before the liquid is absorbed, said difference in level between the convex portions and the concave portions becomes 3A mm or more after the liquid is absorbed.

3. An absorbent sheet according to either one of claims 1 and 2, wherein said difference in level between the convex portions and the concave portions (A) is in a range of from 0.2 mm to 2.0 mm before the liquid is absorbed.

4. An absorbent sheet according to either one of claims 1 through 3, wherein said absorbent sheet comprises a P sheet where an absorbent layer is formed containing super absorbent polymer (SAP) on one side of a substrate sheet and the content of said SAP in said absorbent layer is in a range of from 50 wt% to 95 wt%.

5. An absorbent sheet according to claim 4, wherein the thickness of said P sheet is 2.0 mm or less before the liquid is absorbed.

6. An absorbent sheet according to either one of claims 4 and 5, wherein said P sheet has absorbent layers formed on one side of said substrate sheet in a band form at certain intervals.

7. An absorbent sheet according to either one of claims 4 through 6, wherein a Q sheet is further laminated with said P sheet to hold and fix said P sheet.

8. An absorbent sheet according to claim 7, wherein said P sheet and said Q sheet are bonded intermittently to each other at certain intervals.

9. An absorbent sheet according to claim 8, wherein the interval of bonding points between said P sheet and said Q sheet is 5 mm or more.

10. An absorbent sheet according to either one of claims 7 through 9, wherein said P sheet is bonded to said Q sheet in the area where no absorbent layers are formed.

11. An absorbent sheet according to either one of claims 1 through 10, wherein said convex and concave portion structure is formed with said absorbent sheet folded.

12. An absorbent sheet according to either one of claims 7 through 10, wherein said convex and concave portion structure is formed with said P sheet being bonded to said Q sheet intermittently at certain intervals in such a condition that said P sheet is slackened.

13. An absorbent sheet according to claim 12, wherein said P sheet is bonded to said Q sheet in the area where no absorbent layers are formed.

14. An absorbent sheet according to either one of claims 12 and 13, wherein said P sheet is laminated with and bonded intermittently to at least one layer of an absorbent sheet comprising said substrate sheet with absorbent layers containing said SAP formed thereon so that a laminated structure is formed and said P sheet and said Q sheet are bonded to each other intermittently.

15. An absorbent sheet according to either one of claims 4 through 14, wherein said absorbent layers contain super absorbent polymer (SAP) and microfibrillated cellulose (MFC) as its bonding agent.

16. An absorbent sheet according to either one of claims 4 through 14, wherein said substrate sheet of said P sheet comprises at least one kind selected from hydrophilic and diffusive wood pulp sheet, rayon-based non-woven fabric or cotton-based non-woven fabric or non-woven fabrics treated to be hydrophilic consisting of polyethylene-based, polypropylene-based; polyethylene-terephthalate-based, or polyvinyl-alcohol-based fibers.

17. An absorbent sheet according to either one of claims 7 through 14, wherein said Q sheet comprises at least one hydrophobic non-woven fabric selected from spun bonded or spun bonded/melt blown/spun bonded composites consisting of polyethylene-based, polypropylene-based, polyethylene-terephthalate-based or polyvinyl-alcohol-based fibers.

18. An absorbent sheet according to either one of claims 7 through 14, wherein said Q sheet comprises a film or an apertured film selected from polyethylene-based, polypropylene-based, polyethylene-terephthalate-based or polyvinyl-alcohol-based film or a composite of a non-woven fabric with any of such films.

19. An absorbent product comprising a liquid permeable top sheet, a liquid impermeable leakage preventing sheet and an absorbent body disposed between both sheets for absorbing and holding a liquid and having a liquid guarding function in its side edge portions and/or in the peripheral edge portions of the front and back bodies, wherein said absorbent body comprises an absorbent sheet having a spontaneous three dimension structure according to either one of claims 1 through 18.

20. An absorbent product according to claim 19, wherein said absorbent body is sectioned into a central portion and both side edge portions, said both side edge portions having a laminated structure with a difference in level in terms of thickness of 1.0 mm or less before the liquid is absorbed as compared with said central portion and said both side edge portions being made to stand up by absorbing the liquid to swell so as to have a difference in level in terms of thickness of 2.0 mm or more so that said both side edge portions function as a side guard bank.

21. An absorbent product according to claim 19, wherein said absorbent body is sectioned into a central portion and both side edge portions, said both side edge portions having a laminated structure with a difference in level in terms of thickness of 2.0 mm or less before the liquid is absorbed as compared with said central portion and said both side edge portions being made to stand up by absorbing the liquid to swell so as to have a difference in level in terms of thickness of 5.0 mm or more so that said both side edge portions function as a three-dimensional side guard bank.

22. An absorbent product according to claim 19, wherein said absorbent body is sectioned into its central portion and the peripheral edge portions of its front and back bodies, said peripheral edge portions having a laminated structure with a difference in level in terms of thickness of 1.0 mm or less before the liquid is absorbed as compared with said central portion and said edge portions being made to stand up by absorbing the liquid to swell so as to have a difference in level in terms of thickness of 2.0 mm or more so that said end edge portions function as an end guard bank.

23. An absorbent product according to claim 19, wherein said absorbent body is sectioned into its central portion and the peripheral edge portions of its front and back bodies, said peripheral edge portions having a laminated structure with a difference in level in terms of thickness of 2.0 mm or less before the liquid is absorbed as compared with said central portion and said end edge portions being made to stand up by absorbing the liquid to swell so as to have a difference in level in terms of thickness of 5.0 mm or more so that said end edge portions function as an end guard bank.

24. An absorbent product according to either one of claims 19 through 23, wherein the back body of said absorbent body has an excrement guiding portion formed comprising a convex and concave portion structure with a difference in level in terms of thickness of 2 mm or less before the liquid is absorbed and with a difference in level of 5 mm or more after it absorbs the liquid to swell.

25. An absorbent product according to either one of claims 19 through 24, wherein the longitudinal central region of said absorbent body is made to stand up as it has a difference in level in terms of thickness of 2 mm or less as compared with the peripheral area of said absorbent body before the liquid is absorbed and as it has a difference in level in terms of thickness of 5 mm or more compared with the peripheral area after it absorbs the liquid to swell whereby a convex portion structure is formed that the central region is made to be closely in contact with the discharging area.
